(19) Europäisches Patentamt
European Patent Office
Office européen des brevets

(11) **EP 3 063 294 B1**

(12) **EUROPEAN PATENT SPECIFICATION**

(45) Date of publication and mention
of the grant of the patent:
**05.12.2018 Bulletin 2018/49**

(21) Application number: **14793824.5**

(22) Date of filing: **27.10.2014**

(51) Int Cl.:
**C12Q 1/68** (2018.01)

(86) International application number:
**PCT/EP2014/072953**

(87) International publication number:
**WO 2015/063008 (07.05.2015 Gazette 2015/18)**

(54) **PREDICTION OF HYBRID TRAITS**

VORHERSAGE VON HYBRIDEN MERKMALEN

PRÉDICTION DE TRAITS HYBRIDES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.10.2013 DE 102013111980**

(43) Date of publication of application:
**07.09.2016 Bulletin 2016/36**

(73) Proprietor: **Hi Fidelity Genetics LLC Wilmington, DE 19801 (US)**

(72) Inventors:
• **SCHOLTEN, Stefan**
  **22609 Hamburg (DE)**
• **THIEMANN, Alexander**
  **25474 Bönningstedt (DE)**
• **SEIFERT, Felix**
  **22043 Hamburg (DE)**
• **FRISCH, Matthias**
  **35435 Wettenberg (DE)**
• **MELCHINGER, Albrecht**
  **70599 Stuttgart (DE)**

(74) Representative: **Carpmaels & Ransford LLP One Southampton Row London WC1B 5HA (GB)**

(56) References cited:
**WO-A2-2007/113532**

• **STEFAN SCHOLTEN ET AL: "Transcriptome-Based Prediction of Heterosis and Hybrid Performance", DIAGNOSTICS IN PLANT BREEDING,, 1 January 2013 (2013-01-01), pages 265-279, XP009181930,**
• **JUNJIE FU ET AL: "Partial least squares regression, support vector machine regression, and transcriptome-based distances for prediction of maize hybrid performance with gene expression data", THEORETICAL AND APPLIED GENETICS ; INTERNATIONAL JOURNAL OF PLANT BREEDING RESEARCH, SPRINGER, BERLIN, DE, vol. 124, no. 5, 19 November 2011 (2011-11-19), pages 825-833, XP035019317, ISSN: 1432-2242, DOI: 10.1007/S00122-011-1747-9**
• **M. GROSZMANN ET AL: "Changes in 24-nt siRNA levels in Arabidopsis hybrids suggest an epigenetic contribution to hybrid vigor", PROCEEDINGS OF THE NATIONAL ACADEMY OF SCIENCES, vol. 108, no. 6, 8 February 2011 (2011-02-08), pages 2617-2622, XP55159027, ISSN: 0027-8424, DOI: 10.1073/pnas.1019217108**
• **FANGFANG CHEN ET AL: "Expression Analysis of miRNAs and Highly-expressed Small RNAs in Two Rice Subspecies and their Reciprocal Hybrids", JOURNAL OF INTEGRATIVE PLANT BIOLOGY, vol. 52, no. 11, 26 November 2010 (2010-11-26), pages 971-980, XP55159032, ISSN: 1672-9072, DOI: 10.1111/j.1744-7909.2010.00985.x**

**Description**

The invention relates to a method for the prediction of the amount of heterosis in plants, as defined in the claims.

A hybrid is defined as the offspring of a cross between two parents, usually inbred lines, with different genetic background. Inbred lines which are for example used in commercial hybrid maize breeding (Duvick & Cassman 1999. Post-green revolution trends in yield potential of temperate maize in the north-central United States. Crop Sci. 39: 1622-1630) are individuals without heterozygosity which are produced by constant backcrossing or as doubled haploids by means of biotechnological techniques (Geiger & Gordillo 2009. Doubled haploids in hybrid maize breeding. Maydica 54: 485-499).

Many animal and plant species, when they are grown as hybrids and produced by crossing two genetically different parents, reveal increased growth rates, produce a larger biomass and provide in the case of crops and farm animals higher yield and productivity. This phenomenon is known as hybrid vigor or heterosis (Shull 1908. The composition of a field of maize. American Breeders' Association 4: 296-301) and can be related to almost all aspects of biology and all characteristics of plants and animals when a hybrid exceeds parents.

The extent of heterosis in plants and animals may vary greatly and is estimated as increase compared to the mean value of the two parents (mid-parent heterosis, MPH) or as the increase compared to the parent with the higher performance (best-parent heterosis, BPH).

Heterosis is of enormous importance in many crops as well as in plant and animal breeding, and the production of hybrids having a high extent of heterosis is desirable in breeding (Duvick 1986. Plant breeding: past achievements and expectations for the future. Econ. Bot. 40: 289-297). Despite intensive genetic analyses, the molecular basis of the phenomenon heterosis is not fully understood. To explain the benefits resulting from complementation, combination, or interaction of two different alleles in hybrids, the non-exclusive genetic models of dominance (Bruce 1910. The Mendelian theory of heredity and the augmentation of vigor. Science 32: 627-628; Davenport 1908. Degeneration, albinism and inbreeding. Science 28: 454-455), overdominance (Shull 1908. The composition of a field of maize. American Breeders' Association 4: 296-301; East 1936. Heterosis. Genetics 21: 375-397) and epistasis (Stuber 1994. Heterosis in plant breeding. Plant Breed Rev 12: 227-251; Goodnight 1999. Epistasis and heterosis. In: Coors JG, Pandey S, editors. The Genetics and Exploitation of Heterosis in Crops. Madison: American Society of Agronomy, pp. 59-67) are used. Also, hypothetical models were set up explaining these interactions by gene regulatory networks (Omholt et al. 2000. Gene regulatory networks generating the phenomena of additivity, dominance and epistasis. Genetics 155: 969-980). Nevertheless, in particular, the mechanisms of heterosis of complex, quantitative traits such as yield or vegetative growth, are little understood (Birchler et al. 2010. Heterosis. Plant Cell 22: 2105-2112).

The genetic explanations in the proposed models for, at least, a part of the heterosis observed in hybrids do not provide quantitative information about the extent of heterosis.

Therefore, aside from the genetic explanations, attempt was made to characterize heterosis at the molecular level. The assumption was that quantitative differences in the mRNA pool of certain genes between parents and their hybrids contribute to the molecular basis of heterosis. Such differential gene expression in hybrids relative to the inbred parents could be observed in the past in a variety of studies. These studies showed extensive transcriptome changes in hybrids compared to their parents (Stupar et al. 2008. Gene expression analyses in maize inbreds and hybrids with varying levels of heterosis. BMC Plant Biol. 8: 33; Guo et al. 2006. Genome-wide transcript analysis of maize hybrids: allelic additive gene expression and yield heterosis. Theor. Appl. Genet. 113: 831-845; Swanson-Wagner et al. 2006. All possible modes of gene action are observed in a global comparison of gene expression in a maize F1 hybrid and its inbred parents. Proc. Natl. Acad. Sci. U S A 103: 6805-6810; Meyer et al. 2007. Heterosis associated gene expression in maize embryos 6 days after fertilization exhibits additive, dominant and overdominant pattern. Plant Mol. Biol. 63: 381-391; Jahnke et al. 2010. Heterosis in early seed development: a comparative study of F1 embryo and endosperm tissues 6 days after fertilization. Theor. Appl. Genet. 120: 389-400; Uzarowska et al. 2007. Comparative expression profiling in meristems of inbred-hybrid triplets of maize based on morphological investigations of heterosis for plant height. Plant Mol. Biol. 63: 21-34; Stupar & Springer 2006. Cis-transcriptional variation in maize inbred lines B73 and Mo17 leads to additive expression patterns in the F1 hybrid. Genetics 173: 2199-2210) which were either additive or non-additive. An additive gene expression describes a hybrid expression according to the average of the parental gene expression. An additive hybrid expression can be explained by a differential cis-regulated gene expression. In a non-additive expression, the hybrid expression differs from the average expression of both parents, suggesting a differential contribution of trans-factors (Wittkopp et al. 2004. Evolutionary changes in cis and trans gene regulation. Nature 430: 85-88).

Therefore, studies assume that both differentially cis regulated, additively expressed genes are involved (Guo et al. 2004. Allelic variation of gene expression in maize hybrids. Plant Cell 16: 1707-1716; Springer & Stupar 2007. Allelic variation and heterosis in maize: How do two halves make more than a whole? Genome Res. 17: 264-275; Thiemann et al. 2010. Correlation between parental transcriptome and field data for the characterization of heterosis in Zea mays L. Theor. Appl. Genet. 120: 401-413), as well as differentially trans regulated, non-additive genes. Possible trans effects that may play a role are small RNAs which may affect, among other things, the epigenome (Ha et al. 2009. Small RNAs serve as a genetic buffer against genomic shock in Arabidopsis interspecific hybrids and allopolyploids. Proc. Natl. Acad. Sci. U.S.A. 106: 17835-17840). Changes in the epigenome, at the level of DNA methylation, were observed between hybrids and inbred parents of *Arabidopsis* and rice (Groszmann et al. 2011. Changes in 24-nt siRNA levels in Arabidopsis hybrids suggest an epigenetic contribution to hybrid vigor. Proc. Natl. Acad. Sci. U.S.A., 108: 2617-2622; He et al. 2010. Global Epigenetic and Transcriptional Trends among Two Rice Subspecies and Their Reciprocal Hybrids. Plant Cell 22: 17-33). A link between DNA methylation patterns and gene expression in hybrids was observed in rice (Chodavarapu et al. 2012. Transcriptome and methylome interactions in rice hybrids. Proc. Natl. Acad. Sci. U.S.A. 109: 12040-12045). In maize and sugar beet it could be shown that changes in DNA methylation are not limited to transposons, but also occur in gene coding regions (Zhao et al. 2007. Epigenetic inheritance and variation of DNA methylation level and pattern in maize intra-specific hybrids. Plant Science 172: 930-938; Zhang et al. 2007. Endosperm-specific hypomethylation, and meiotic inheritance and variation of DNA methylation level and pattern in sorghum (Sorghum bicolor L.) inter-strain hybrids. Theor. Appl. Genet. 115: 195-207).

Small RNAs are closely linked to the DNA methylation (Lister et al. 2008. Highly integrated single-base resolution maps of the epigenome in Arabidopsis. Cell 133:523-36). They contribute to genome stability and are important regulators of gene expression (Van Wolfswinkel & Ketting 2010. The role of small non-coding RNAs in genome stability and chromatin organization. J. Cell Sci. 2010 123: 1825-39). Small RNAs are 15-nt to 40-nt long and either cause a transcriptional gene silencing (TGS) or a post transcriptional gene silencing (PTGS). Depending on the different biosynthetic pathways, they are devided in siRNAs (small interfering RNAs) and miRNAs (micro RNAs) (Bartel 2004. MicroRNAs: Genomics, biogenesis, mechanism, and function. Cell 116: 281-297). miRNAs are derived from so-called MIR genes encoding single stranded transcripts which then form a hairpin structure and are subsequently processed into mature miRNAs by dicer proteins (Bartel 2004. MicroRNAs: Genomics, biogenesis, mechanism, and function. Cell 116: 281-297). siRNAs, however, result from double stranded RNAs and have a wide range of places of origin and/or biogenesis. Double stranded RNAs which can lead to the formation of siRNAs are derived e.g. from of reverse complementary sequence regions (inverted repeat) of transcribed RNA, natural cis-antisense transcript pairs, RNA dependent RNA polymerases (RDRs), the replication of RNA viruses or retro element rich genome regions (Khraiwesh et al. 2012. Role of miRNAs and siRNAs in biotic and abiotic stress responses of plants. Biochim. Biophys. Acta 1819: 137-48). Double stranded RNAs are processed via dicer into short double stranded siRNAs. They are, inter alia, important repressors of transposons and viral sequences (Mallory & Vaucheret 2006. Functions of microRNAs and related small RNAs in plants. Nat. Genet. 38: S31-S36).

First evidence for an involvement of small RNAs in the heterosis effect came from maize and Arabidopsis and showed a differential expression of small RNAs between hybrids and inbred lines (Barber et al. 2012. Repeat associated small RNAs vary among parents and following hybridization in maize. Proc. Natl. Acad. Sci. U.S.A., 106: 10444-10449; Groszmann et al. 2011. Changes in 24-nt siRNA levels in Arabidopsis hybrids suggest an epigenetic contribution to hybrid vigor. Proc. Natl. Acad. Sci. U.S.A. 108: 2617-2622) . In these studies, 24-nt siRNAs were reduced in the hybrids and regulatory miRNAs non-additively expressed. The reduced siRNAs were mostly associated with regulatory genes and their flanking regions and also showed an association with the gene expression in hybrids. It has been suggested that this so-called epigenetically regulated epi-alleles could contribute to heterosis via the hybrid variation (Groszmann et al. 2011. Changes in 24-nt siRNA levels in Arabidopsis hybrids suggest an epigenetic contribution to hybrid vigor. Proc. Natl. Acad. Sci. U.S.A. 108: 2617-2622). In maize, a certain class of differentially expressed 22-nt long siRNAs has been identified (Nobuta et al. 2008. Distinct size distribution of endogeneous siRNAs in maize: Evidence from deep sequencing in the mop1-1 mutant. Proc. Natl. Acad. Sci. U.S.A.105: 14958-14963). A study in two inbred lines and their reciprocal hybrids revealed that the 22-nt siRNAs differentially expressed between inbred lines and hybrids originate from certain retrotransposons and could contribute to heterosis by virtue of variability (Barber et al. 2012. Repeat associated small RNAs vary among parents and following hybridization in maize. Proc. Natl. Acad. Sci. U.S.A. 106: 10444-10449). A further study with reciprocal hybrids and their parents in maize revealed complex epigenetic alterations in hybrids compared to their parents based on DNA methylation, histone modification and small RNAs (Shen et al. 2012. Genome-Wide Analysis of DNA Methylation and Gene Expression Changes in Two Arabidopsis Ecotypes and Their Reciprocal Hybrids. The Plant Cell 24: 875-892). Scholten et al. hypothesize that small non-coding RNAs have a prediction potential (Diagnostics in plant breeding (20130101) pages 265ff).

But the observations made so far give no indication of a direct and quantitative influence of small RNAs with respect to heterosis.

The production of hybrids with strong heterosis is currently made primarily on the basis of trial and error in field trials. For that purpose, genetically different parents are crossed and the offsprings are grown to test their characteristics (Windhausen et al. 2012. Effectiveness of genomic prediction of maize hybrid performance in different breeding populations and environments. G3 2: 1427-1436). As important traits such as yield can be measured only late in the life cycle, these tests are very time consuming. In addition, the genetic distance between two parents in some cases has an inconsistent correlation to heterosis and therefore is insufficient for predicting hybrid performance (Melchinger 1999.

Genetic diversity and heterosis. In: Coors JG, Pandey S (eds) The genetics and exploitation of heterosis in crops. ASA-CSSA, Madison, p 99-118). Due to these limitations, many not suitable hybrids are examined in the course of breeding which results in high costs. In particular by the large number of inbred lines which are now produced in commercial hybrid breeding programs in each breeding cycle, the possible hybrids can not be tested completely due to the high cost of field trials, leading to significant losses of potentially outstanding crossing partners (Schrag et al. 2006. Prediction of single-cross hybrid performance for grain yield and grain dry matter content in maize using AFLP markers associated with QTL. Theor. Appl. Genet. 113: 1037-1047).

Methods which allow a prediction of hybrid traits have been developed on the basis of genetic markers that represent polymorphisms of the DNA sequence between the parent lines (Schrag et al. 2006. Prediction of single-cross hybrid performance for grain yield and grain dry matter content in maize using AFLP markers associated with QTL. Theor. Appl. Genet. 113: 1037-1047; Schrag et al. 2009. Molecular marker-based prediction of hybrid performance in maize using unbalanced data from multiple experiments with factorial crosses. Theor. Appl. Genet. 118: 741-751). The accurate and reliable prediction on the basis of genetic markers remains a challenge (Windhausen et al. 2012. Effectiveness of Genomic Prediction of Maize Hybrid Performance in Different Breeding Populations and Environments. G3 2: 1427-1436; Reif et al. 2012. Genomic prediction of sunflower hybrid performance. Plant Breed. 132: 107-114). Genetic markers were also used in combination with metabolite data of parent lines for the prediction of hybrid traits (Gärtner et al. 2009. Improved Heterosis Prediction by Combining Information on DNA- and Metabolic Markers. PLOS ONE 4: e5220; Riedelsheimer et al. 2012. Genomic and metabolic prediction of complex heterotic traits in hybrid maize. Nature Publishing Group 44: 217-220). These experimental models to integrate multiple levels of data show the high demand and the great interest in improving the accuracy and reliability of methods for the prediction of hybrid traits.

From studies where it was observed that gene expression is altered in hybrids compared to the parent lines, it was concluded that hybrid effects are associated with altered gene expression patterns (Hochholdinger & Hoecker 2007. Towards the molecular basis of heterosis. Trends in Plant Science 12: 427-432). Predictions of the hybrid performance and heterosis could be made on the basis of parental transcription profiles in maize (Fu et al. 2012. Partial least squares regression, support vector machine regression, and transcriptome-based distances for prediction of maize hybrid performance with gene expression data. Theor. Appl. Genet. 124: 825-833; Frisch et al. 2010. Transcriptome-based distance measures for grouping of germplasm and prediction of hybrid performance in maize. Theor. Appl. Genet. 120: 441-450) and Arabidopsis (Stokes et al. 2010. An association transcriptomics approach to the prediction of hybrid performance. Mol. Breeding 26: 91-106).

Moreover, for tomato (Shivaprasad et al. 2011. Extraordinary transgressive phenotypes of hybrid tomato are influenced by epigenetics and small silencing RNAs. The EMBO Journal 31: 257-266), Arabidopsis (Groszmann et al. 2011. Changes in 24-nt siRNA levels in Arabidopsis hybrids suggest an epigenetic contribution to hybrid vigor. Proc. Natl. Acad. Sci. U.S.A. 108: 2617-2622), maize (Barber et al. 2012. Repeat associated small RNAs vary among parents and following hybridization in maize. Proc. Natl. Acad. Sci. U.S.A. 109: 10444-10449) and rice (Chodavarapu et al. 2012. Transcriptome and methylome interactions in rice hybrids. Proc. Natl. Acad. Sci. U.S.A. 109: 12040-12045) it was recently mentioned that the expression of small RNAs differs between parents and their hybrids. In particular, the differences in the expression of 24-nt sRNAs led to the hypothesis of an epigenetic component of heterosis.

However, all previously known methods are not applicable to predict the heterosis or performance of a specific hybrid quantitatively and thus to determine the most promising parent lines. A method that allows, on the basis of the parent lines, an accurate prediction of the amount of heterosis or hybrid vigor of the resulting hybrids, is not yet known. The object of the present invention is therefore to provide such a method which enables, on the basis of the parent lines, an accurate prediction of the amount of heterosis or hybrid vigor of the resulting hybrids.

According to the invention the object is achieved by a method, wherein sRNA molecules which are associated with a hybrid trait are identified and parent lines which are suitable for the production of hybrids are analyzed for the level of an expression of the identified sRNA molecules; wherein the identification of the sRNA molecules comprises the steps of:

    a) determination of the extent of the expression of traits in different hybrids produced from genetically different parent lines; and
    b) analysis of the parent lines of the tested hybrids in terms of their sRNA expression.

Thus, the invention allows the selection of suitable organisms for the production of plant hybrids having an increased hybrid vigor respectively heterosis for one or more hybrid traits, such as yield, fertility, stress resistance, etc.

The method according to the invention, as defined in the claims, can start with previously identified sRNA molecules so that the most promising parent lines are selected on the basis of the expression of these sRNA molecules. On the other hand, it is possible to identify new sRNA molecules. This is necessary for example, if for certain organisms no corresponding sRNA molecules were described or organisms have not been tested for a particular trait so far.

A preferred embodiment of the method, therefore, provides for the identification of sRNA molecules comprising the steps of:

 a) cultivation of plants or animals of genetically different parent lines;
 b) crossing said plants or animals for the production of hybrids;
 c) determination of the extent of the expression of traits in different hybrids;
 d) analysis of the parent lines of the tested hybrids in terms of their sRNA expression;

The analysis of the parent lines in terms of the expression level of the identified sRNA molecules is carried out, for example, by determining the number of sRNA molecules. Hereby, the differential sRNA expression between genetically different parent lines is determined.

Surprisingly, it was found that expression profiles of so-called small RNAs (sRNAs) having a length of 15 to 40 nucleotides, which do not encode proteins, allow conclusions about hybrid traits. In particular, these sRNAs can be used to make predictions about the extent of heterosis or other characteristics of hybrids resulting from the crossing of plants or animals. Thus, this invention provides significant benefits for the breeding process, because predictions regarding hybrid traits can be made already a generation in advance without collecting field data for the actual genotypes. Compared to a method using mRNA expression profiles, the present invention is distinguished by a particularly high accuracy of prediction.

Measurements of the sRNA expression in individual organisms or in a group of organisms, such as inbred lines or hybrids of a factorial crossing scheme of a breeding population, are preferably carried out using high-throughput sequencing (e.g. pyrosequencing, sequencing by hybridization, ion semiconductor DNA sequencing, sequencing by bridge synthesis, two base sequencing, paired-end sequencing) or high-throughput sequencing by means of measuring the reaction of individual molecules (e.g. protons, fluorophores) or conventional methods (e.g. method of Maxam and Gilbert (Maxam & Gilbert 1977. A new method for sequencing DNA. Proc. Natl. Acad. Sci. U.S.A., 74: 560-564) Sanger's dideoxy method (Sanger & Coulson 1975. A rapid method for determining sequences in DNA by primed synthesis with DNA polymerase. Journal of Molecular Biology, 94: 441-448)), in order to comprehensively as possible record the existing individual sRNAs quantitatively. Shall a predetermined set of sRNAs be used for prediction, the sRNA expression data can be measured using PCR methods (e.g. quantitative RT-PCR (Varkonyi-Gasic et al. 2007. Protocol: a highly sensitive RT-PCR method for detection and quantification of microRNAs. Plant Methods 3: 12)) or microarray experiments (Bowtell & Sambrook 2003. DNA microarrays: a molecular cloning manual. Cold Spring Harbor Laboratory Press, Cold Spring Harbor, N.Y.).

The sRNA expression data should preferably be normalized for optimal comparability between different measurements. For this purpose various methods can be used. For expression data from sequencing, expression values can be adjusted e.g. by a scaling factor as the sequencing depth. Alternatively, a normalization can be made by endogenous or artificial spike-in controls.

Other possible methods are quantile, mean, or variance based normalization (McCormick et al. 2011. Experimental design, preprocessing, normalization, and differential expression analysis of small RNA sequencing experiments. Silence 2: 2).

Characteristic data used for association with sRNA expression data can be obtained qualitatively and/or quantitatively from field/greenhouse experiments and laboratory experiments or during the breeding or production process. Qualitative and/or quantitative characteristic data can be obtained from inbred lines, hybrids, transgenic or other genotypes.

The correlation analysis of sRNA data with characteristic data can be performed using either a pre-defined fixed set of sRNAs or an association study hybrids specific set of sRNAs.

The association of sRNA expression data with qualitative characteristic data is preferably performed via a binomial probability test. Individuals are split into two or more qualitative groups related to the characteristic, and the probability of a non-random association of certain sRNA expression patterns is analyzed (see Example 1). sRNA expression patterns can be, for example, differential expressions between different individuals (e.g. hybrid parents) as well as differences of absolute expression values. The identification of differentially expressed sRNAs can be achieved by defining a minimum expression and a minimum relative and/or absolute difference in expression. Alternatively, a differential expression can be established by statistical tests (e.g. F-test, t-test, Anova). Biological and/or technical replicates are preferably used.

The association of sRNA expression data with quantitative characteristic data can be performed as described for qualitative characteristic data so that the quantitative characteristic values are preferably split into qualitative classes (e.g. low and high characteristic values). Alternatively to a binomial probability test, associated sRNAs can be identified by means of parametric/non-parametric regression methods (e.g. linear regression) or other mathematical methods of pattern recognition (e.g. SVM, Random Forest, Neural Networks).

In addition to the quantitative consideration of expression data of individual sRNAs for association with characteristic data, these expression data can also be integrated on the basis of certain criteria (e.g. genomic sequence regions/annotations). A further possibility is the purely qualitative consideration of sRNAs (e.g. expression over a certain level) for association with characteristic data via e.g. a binomial probability test.

The prediction of characteristic data can be made using sRNA expression data of an individual or on the basis of expression data of the parents of an offspring. For a prediction, sRNA expression and characteristic data of other individuals (for individuals characteristic prediction) or sRNA expression data of parents and characteristic data of its offsprings must exist to a limited extent. The prediction is carried out by determining prediction parameters (e.g. regression parameters) based on known individuals and applying these parameters on the sRNA expression data of the individual having the trait to be predicted.

The prediction parameters can be determined based on the absolute sRNA expression data, or alternatively via distance measures (e.g. between parents of an offspring or individuals and a reference individual).

In summary, the invention comprises the use of sRNA expression analyzes of plants, or their hybrids and/or inbred lines or any other genotypes, as defined in the claims, (1) to make predictions about the extent of heterosis and other traits in plants or animals, and (2) to identify crossing partners which provide advantageous combinations with respect to their sRNA profiles and the offsprings of which provide improved characteristics with respect to one or more traits. Thus, for example, a set of 11,272 sRNAs can be identified which allows the prediction of heterosis for grain yield in maize and also can be used as a complete set or in part for the prediction of analogous characteristics in other plant species.

The invention allows the breeder a prediction of heterosis of different traits and the prediction of non-heterotic characteristics by examining the sRNA expression profiles. Also the invention allows the prediction of characteristics by the analysis of very early stages (e.g. seedling) of the same or the next generation. The sRNA sample material which is used to generate the sRNA expression data may differ with respect to the developmental stage or the tissue of the expressed characteristics. This means that the tissues used for the investigation of sRNAs must not be identical with those of the characteristics measurement. This results in less time and money spent for the cultivation as well as for the entire selection process in the breeding. Furthermore, the use of early development stages allows controlled culture conditions (e.g. greenhouse, growth chamber) and thus a reproducible prediction. In case of plant breeding, field trials can be avoided by the testing of seedlings of parent lines or parental genotypes to predict offsprings.

For the purposes of this application, plants include for example, cereals such as wheat, barley, rice or maize, vegetables such as paprika, onions, carrots or tomatoes, fruit plants such as apple, pear, cherry or wine and other economically relevant plants such as legumes, grasses (e.g. Miscanthus) or algae for biomass production or trees for timber (e.g. poplar).

A requirement for heterosis in plants is the breeding of inbred lines or low heterozygous lines for the production of hybrids. This is easiest in plants with a naturally occurring allogamy (cross-pollination). There is also the possibility to use the male sterility which is divided into the so-called "Genic (nuclear) Male Sterility" (GMS) and the "Cytoplasmic Male Sterility" (CMS) (Bruce 1910. The Mendelian theory of heredity and the augmentation of vigor. Science 32: 627-628).

Other applications of the disclosure include e.g. animals, except humans, such as mammals, birds and fish. Examples in which heterosis plays an important role include farm animals such as cattle, pigs, sheep, goats, chickens or turkeys. Fish from fish farming in which heterosis is of importance, include e.g. salmon or carp. It is also conceivable to apply the disclosure to agriculturally non relevant breeding animals such as racing horses or dogs. For the invention described here, a direct and quantitative association of sRNAs with heterosis or other characteristics is used for prediction.

Studies also revealed large differences in sRNA expression profiles between different inbred lines and between inbred lines and their hybrid offsprings. The investigations of sRNA populations of several inbred lines and of the corresponding extensive field data allows to determine a direct impact of certain sRNAs on heterosis.

The investigation of a total of 21 inbred lines of two heterotic groups of maize (hard and dent maize) led to the aforementioned identification of 11,272 sRNAs whose differential parental expression is associated with the heterosis for grain yield. Of these, 6,915 are negative and 4,357 are positive associated with the heterosis for grain yield.

Heterosis in maize hybrids can thus be successfully predicted on the basis of differential parental sRNA expression. Because heterosis is a widespread phenomenon which is not limited to maize or other grains, but also occurs in animals, a use of sRNAs described here as a complete set or in part is quite conceivable in other plant and animal species, provided that these are conserved and a similar heterotic trait compared to the grain yield shall be predicted. Otherwise, the method according to the invention requires the identification of new sets of predictive sRNAs by an association of sRNA expression data and the respective characteristic data of the parents and hybrids. With these predictive sRNAs a heterosis prediction is possible for other crossing partners.

The invention described here and as defined in the claims is complementary to other methods for the prediction of hybrid characteristics and can be combined in integrated models with different levels of data, such as genetic markers (e.g. SNPs and other DNA markers), mRNA, protein, or metabolite data (Riedelsheimer et al. 2012. Genomic and metabolic prediction of complex heterotic traits in hybrid maize. Nature Publishing Group 44: 217-220).

The present invention also relates to the use of sRNA molecules for the prediction of hybrid traits in plants, as defined in the claims. The sRNA molecules are, in particular, differentially expressed parental sRNA molecules which are associated with the extent of the expression of the hybrid trait. The number of sRNA molecules allows a good prediction of the extent of heterosis.

Example

    Figure 1: Linear regression of the combined binary distances $D_b$ of 98 hybrids
    Figure 2: Accuracy of sRNA based prediction of heterosis for grain yield

The sRNA expression of maize inbred lines from the two heterotic groups hard and dent maize was measured. Maize is suitable for these studies due to the comparatively large range of heterosis levels.

A total of 21 inbred lines of a 7x14 crossing scheme were grown under controlled conditions. Four of the seven hard maize lines had a European flint, the remaining three a flint/Lancaster genetic background. Eight of the dent maize lines had an Iowa Stiff Stalk Synthetic and 6 had an Iodent genetic background. The field data of the 98 hybrids from the crossing scheme and those of the inbred lines were obtained from different locations in Germany. The field trials were carried out with a 2 row experimental arrangement with 2-3 biological replicates. Grain yield was measured in Mg/ha at 155 g/kg grain moisture. Heterosis for each inbred line pair and their hybrid was determined as MPH (mean parental heterosis) in Mg/ha at 155 g/kg grain moisture.

Five biological replicates of seedlings of each inbred line were pooled 7 days after sowing and RNA was isolated from the total biological material. The sRNAome was analyzed by means of illumina deep sequencing.

Sequencing adapters were eliminated from the raw sequencing data and sequence regions having a low sequencing quality below 99.9% were removed. All redundant sequences with a length of 15-nt to 40-nt were combined for the following analysis and their sequence number was determined (sRNA expression).

The sRNA expression data were quantile normalized with a modification preventing the allocation of normalized expression data to unexpressed sRNAs (Bolstad et al. 2003. A comparison of normalization methods for high density oligonucleotide array data based on variance and bias. Bioinformatics 19: 185-93).

The quantile normalized expression values were normalized to a sequence number per million sequences per sequencing library (read count per million quantile normalized reads, rpmqn). This allows the comparison of different sequence libraries with different sequencing depth.

sRNAs having a minimal expression of 0.5 rpmqn were assumed to be expressed by definition. Differentially expressed were sRNAs with a minimum difference in expression by a factor >= 2 or if one parent was below the minimum expression, the other parent had to have at least the minimum expression multiplied by the minimum expression difference (= 1 rpmqn).

The association of parentally differentially expressed sRNAs with heterosis was based on the method of Frisch et al. (Frisch et al. 2010. Transcriptome-based distance measures for grouping of germplasm and prediction of hybrid performance in maize. Theor. Appl. Genet. 120: 441-450) with the extension to negatively associated sRNAs. The 98 hybrids were, according to their heterosis levels, split into two classes (low/high heterosis) and for each sRNA in both classes the number of hybrids with differential parental expression was determined. For each sRNA $o_l$ and $o_h$ was determined, corresponding to the number of hybrids in the class of low and high heterosis, the parents of which have a differential expression of the examined sRNA. The subsequently calculated binomial distribution probability represents the probability of the differential expression being unequally distributed in two classes, and is thus associated with MPH of grain yield. The probability is calculated according to formula 3 as a function of the number of the differential expression of the sRNA in the defined classes (Formula 1 or 2):

$$k_{min} = o_h, k_{max} = (o_h + o_l) \quad \forall \quad o_l <= o_h \quad (1)$$

$$k_{min} = 0, k_{max} = o_l \quad \forall \quad o_l > o_h \quad (2)$$

$$P_f = \sum_{k=k_{min}}^{k_{max}} Bin_{n,p}(k) \quad with \quad n = (o_h + o_l), p = \tfrac{1}{2} \quad (3)$$

p-values < 0.05 according to Benjamini-Hochberg FDR correction (Benjamini & Hochberg 1995. Controlling the false discovery rate: a practical and powerful approach to multiple testing. J. R. Statist. Soc. B 57: 289-300) indicate a significant association of the tested sRNA with MPH of grain yield. For each hybrid the combined binary distance $D_b$ was determined based on an examination set of sRNAs comprising the number $n_{f,pos}$ corresponding to the positively associated sRNAs and $n_{f,neg}$ corresponding to the negatively associated sRNAs as well as the numbers of differentially expressed sRNAs $n_{d,pos}$ of the positively associated sRNAs and $n_{d,neg}$ of the negatively associated sRNAs from the examination set (Formula 4):

$$D_b = \frac{\sqrt{\frac{n_{d,pos}}{n_{f,pos}}} \cdot n_{f,pos} + \left(1 - \sqrt{\frac{n_{d,neg}}{n_{f,neg}}}\right) \cdot n_{f,neg}}{n_{f,pos} + n_{f,neg}} \qquad (4)$$

From the sRNA expression data of the 21 inbred lines a total of 11,272 sRNAs were identified which are significantly associated with heterosis for grain yield. 4,357 of these sRNAs are positively and 6,915 are negatively associated with heterosis for grain yield.

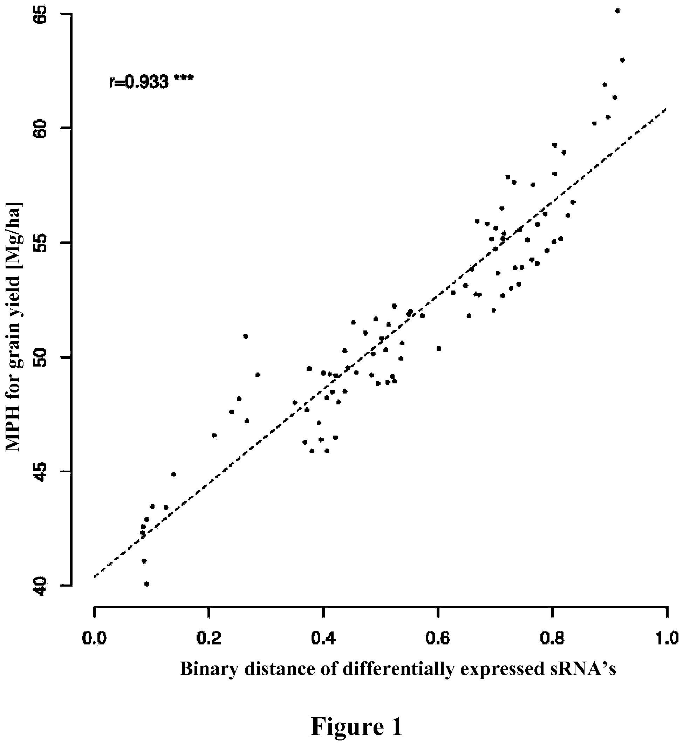

A linear regression of the combined binary distances $D_b$ based on the 11,272 sRNAs of the tested 98 hybrids significantly associated with heterosis for grain yield showed a high correlation of r = 0.933 with the associated trait of heterosis for grain yield (Figure 1).

This high correlation suggests a high prediction accuracy of sRNAs. To confirm this assumption, type-0 and type-2 cross-validations as described by Frisch et al. (Frisch et al. 2010. Transcriptome-based distance measures for grouping of germplasm and prediction of hybrid performance in maize. Theor. Appl. Genet. 120: 441-450) were carried out with a total of 100 executions. For this purpose, two non-overlapping sets of inbred parents were defined in each validation, the determination set through which prediction parameters are determined, and the prediction set for the hybrids of which heterosis for grain yield is to be predicted. For each cross-validation all sRNAs associated with heterosis for grain yield are determined (p < 0.05 without error correction) based on the possible hybrids of the determination set. Based on the determined associated sRNAs of destination set, the combined binary distance $D_b$ can be determined for the parents of the determination set of each of the hybrids. The parameters a (y-axis intercept) and b (slope) of the linear equation (Formula 5) can be calculated for the determination set by means of the least squares method (least square minimization) using the values H and $D_b$ known for the hybrids of the destination set. The calculated prediction parameters (a and b) and the binary distance $D_b$, individually calculated for each hybrid of the prediction set on the basis of associated sRNAs of the destination set, are now used to predict the heterosis for grain yield H for each of these hybrids (Formula 5).

$$H = a + b \cdot D_b \qquad (5)$$

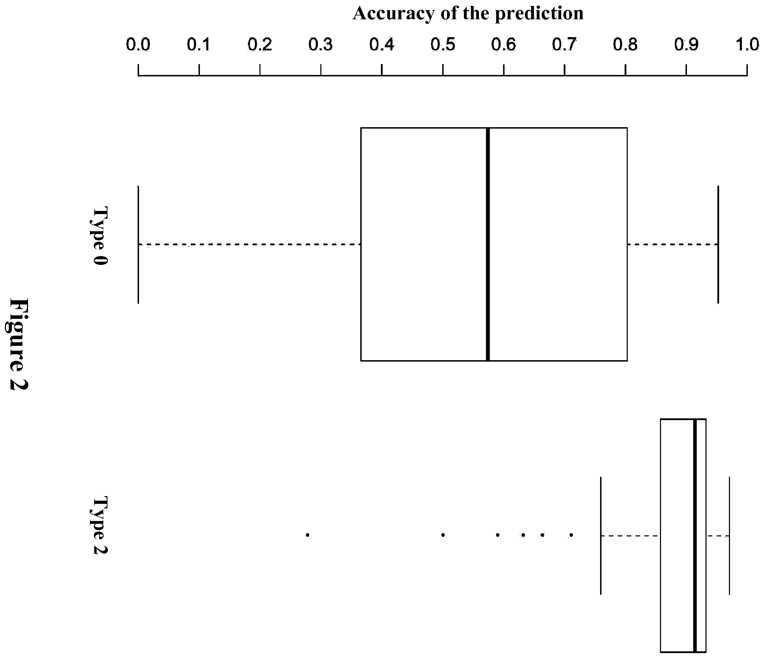

In each cross-validation test, the combined binary distance $D_b$ is calculated for each hybrid of inbred parents pairings of the prediction set according to Formula 4, and the characteristic value for heterosis for grain yield H is calculated by means of the determined regression parameters a and b determined via the destination set, as previously described, according to formula 5. The accuracy of the prediction is determined by means of the correlation coefficients of the predicted and the known characteristic values for heterosis for grain yield of hybrids of the prediction set. The results for type-0 and type-2 cross-validations are shown in Figure 2.

The method according to the present invention allows a high predictive quality for sRNA expression data for heterosis in particular for grain yield.

**Claims**

1. A method for the prediction of the amount of heterosis in plants, wherein sRNA molecules which are associated with a hybrid trait are identified and parent lines which are suitable for the production of hybrids are analyzed for the level of an expression of the identified sRNA molecules; wherein the identification of the sRNA molecules comprises the steps of:

a) determination of the extent of the expression of traits in different hybrids produced from genetically different parent lines; and
b) analysis of the parent lines of the tested hybrids in terms of their sRNA expression.

2. A method according to claim 1, **characterized in that** the sRNA molecules have a length of 15 to 40 nucleotides.

3. A method according to one of claims 1 to 2, **characterized in that** the sRNA molecules do not encode proteins.

4. A method according to one of claims 1 to 3, **characterized in that** a determination of differential sRNA expression is carried out between genetically different parent lines.

5. A method according to one of claims 1 to 4, **characterized in that** the trait is yield.

6. A method according to one of claims 1 to 5, **characterized in that** an analysis of the parent lines regarding its sRNA expression is carried out in plant seedlings.

7. The use of sRNA molecules for the prediction of the amount of heterosis in plants.

8. The use according to claim 7, **characterized in that** the sRNA molecules are differentially expressed parental sRNA molecules which are associated with the extent of the expression of a trait.


**Patentansprüche**

1. Verfahren zum Vorhersagen des Ausmaßes an Heterosis bei Pflanzen, wobei sRNA-Moleküle, welche mit einem Hybridmerkmal in Verbindung stehen, identifiziert werden und Elternlinien, welche sich zur Erzeugung von Hybriden eignen, auf das Expressionsniveau der identifizierten sRNA-Moleküle untersucht werden; wobei die Identifizierung der sRNA-Moleküle die folgenden Schritte umfasst:

   a) Bestimmen des Ausmaßes der Expression von Merkmalen bei verschiedenen Hybriden, die ausgehend von genetisch unterschiedlichen Elternlinien erzeugt wurden; und
   b) Untersuchung der Elternlinien der geprüften Hydride hinsichtlich ihrer sRNA-Expression.

2. Verfahren gemäß Anspruch 1, **dadurch gekennzeichnet, dass** die sRNA-Moleküle eine Länge von 15 bis 40 Nukleotiden haben.

3. Verfahren gemäß einem der Ansprüche 1 bis 2, **dadurch gekennzeichnet, dass** die sRNA-Moleküle nicht für Proteine codieren.

4. Verfahren gemäß einem der Ansprüche 1 bis 3, **dadurch gekennzeichnet, dass** eine Bestimmung des sRNA-Expressionsunterschieds zwischen genetisch unterschiedlichen Elternlinien durchgeführt wird.

5. Verfahren gemäß einem der Ansprüche 1 bis 4, **dadurch gekennzeichnet, dass** es sich bei dem Merkmal um den Ertrag handelt.

6. Verfahren gemäß einem der Ansprüche 1 bis 5, **dadurch gekennzeichnet, dass** eine Untersuchung der Elternlinien hinsichtlich ihrer sRNA-Expression an Pflanzensaatgut vorgenommen wird.

7. Verwendung von sRNA-Molekülen zum Vorhersagen des Ausmaßes an Heterosis bei Pflanzen.

8. Verwendung gemäß Anspruch 7, **dadurch gekennzeichnet, dass** es sich bei den sRNA-Molekülen um unterschiedlich stark exprimierte sRNA-Moleküle der Eltern handelt, welche mit dem Ausmaß der Expression eines Merkmals in Verbindung stehen.


**Revendications**

1. Méthode de prédiction de la quantité d'hétérosis chez des plantes, **caractérisée en ce que** des molécules de sARN qui sont associées à un caractère hybride sont identifiées et les lignées parentales qui sont convenables pour la production d'hybrides sont analysées pour déterminer le taux d'expression des molécules de sARN identifiées ; où l'identification des molécules de sARN comprend les étapes

   a) de détermination de l'étendue de l'expression de caractères chez différents hybrides produits à partir de lignées parentales génétiquement différentes ; et
   b) d'analyse des lignées parentales des hybrides testés en ce qui concerne leur expression de sARN.

2. Méthode selon la revendication 1, **caractérisée en ce que** les molécules de sARN possèdent une longueur allant de 15 à 40 nucléotides.

3. Méthode selon l'une quelconque des revendications 1 à 2, **caractérisée en ce que** les molécules de sARN ne codent pas pour des protéines.

4. Méthode selon l'une quelconque des revendications 1 à 3, **caractérisée en ce qu'**une détermination de l'expression différentielle de sARN est effectuée entre des lignées parentales génétiquement différentes.

5. Méthode selon l'une quelconque des revendications 1 à 4, **caractérisée en ce que** le caractère est le rendement.

6. Méthode selon l'une quelconque des revendications 1 à 5, **caractérisée en ce qu'**une analyse des lignées parentales en ce qui concerne leur expression de sARN est effectuée sur des plantules.

7. Utilisation de molécules de sARN pour la prédiction de la quantité d'hétérosis chez des plantes.

8. Utilisation selon la revendication 7, **caractérisée en ce que** les molécules de sARN sont des molécules de sARN parentales exprimées de manière différentielle qui sont associées à l'étendue de l'expression d'un caractère.

**Figure 1**

**Figure 2**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **DUVICK ; CASSMAN.** Post-green revolution trends in yield potential of temperate maize in the north-central United States. *Crop Sci.,* 1999, vol. 39, 1622-1630
- **GEIGER ; GORDILLO.** Doubled haploids in hybrid maize breeding. *Maydica,* 2009, vol. 54, 485-499
- **SHULL.** The composition of a field of maize. American Breeders' Association, 1908, vol. 4, 296-301
- **DUVICK.** Plant breeding: past achievements and expectations for the future. *Econ. Bot.,* 1986, vol. 40, 289-297
- **BRUCE.** The Mendelian theory of heredity and the augmentation of vigor. *Science,* 1910, vol. 32, 627-628
- **DAVENPORT.** Degeneration, albinism and inbreeding. *Science,* 1908, vol. 28, 454-455
- **EAST.** *Heterosis. Genetics,* 1936, vol. 21, 375-397
- **STUBER.** Heterosis in plant breeding. *Plant Breed Rev,* 1994, vol. 12, 227-251
- Epistasis and heterosis. **GOODNIGHT.** The Genetics and Exploitation of Heterosis in Crops. American Society of Agronomy, 1999, 59-67
- **OMHOLT et al.** Gene regulatory networks generating the phenomena of additivity, dominance and epistasis. *Genetics,* 2000, vol. 155, 969-980
- **BIRCHLER et al.** *Heterosis. Plant Cell,* 2010, vol. 22, 2105-2112
- **STUPAR et al.** Gene expression analyses in maize inbreds and hybrids with varying levels of heterosis. *BMC Plant Biol.,* 2008, vol. 8, 33
- **GUO et al.** Genome-wide transcript analysis of maize hybrids: allelic additive gene expression and yield heterosis. *Theor. Appl. Genet.,* 2006, vol. 113, 831-845
- **SWANSON-WAGNER et al.** All possible modes of gene action are observed in a global comparison of gene expression in a maize F1 hybrid and its inbred parents. *Proc. Natl. Acad. Sci. U S A,* 2006, vol. 103, 6805-6810
- **MEYER et al.** Heterosis associated gene expression in maize embryos 6 days after fertilization exhibits additive, dominant and overdominant pattern. *Plant Mol. Biol.,* 2007, vol. 63, 381-391

- **JAHNKE et al.** Heterosis in early seed development: a comparative study of F1 embryo and endosperm tissues 6 days after fertilization. *Theor. Appl. Genet.,* 2010, vol. 120, 389-400
- **UZAROWSKA et al.** Comparative expression profiling in meristems of inbred-hybrid triplets of maize based on morphological investigations of heterosis for plant height. *Plant Mol. Biol.,* 2007, vol. 63, 21-34
- **STUPAR ; SPRINGER.** Cis-transcriptional variation in maize inbred lines B73 and Mo17 leads to additive expression patterns in the F1 hybrid. *Genetics,* 2006, vol. 173, 2199-2210
- **WITTKOPP et al.** Evolutionary changes in cis and trans gene regulation. *Nature,* 2004, vol. 430, 85-88
- **GUO et al.** Allelic variation of gene expression in maize hybrids. *Plant Cell,* 2004, vol. 16, 1707-1716
- **SPRINGER ; STUPAR.** Allelic variation and heterosis in maize: How do two halves make more than a whole?. *Genome Res.,* 2007, vol. 17, 264-275
- **THIEMANN et al.** Correlation between parental transcriptome and field data for the characterization of heterosis in Zea mays L. *Theor. Appl. Genet.,* 2010, vol. 120, 401-413
- **HA et al.** Small RNAs serve as a genetic buffer against genomic shock in Arabidopsis interspecific hybrids and allopolyploids. *Proc. Natl. Acad. Sci. U.S.A.,* 2009, vol. 106, 17835-17840
- **GROSZMANN et al.** Changes in 24-nt siRNA levels in Arabidopsis hybrids suggest an epigenetic contribution to hybrid vigor. *Proc. Natl. Acad. Sci. U.S.A.,* 2011, vol. 108, 2617-2622
- **HE et al.** Global Epigenetic and Transcriptional Trends among Two Rice Subspecies and Their Reciprocal Hybrids. *Plant Cell,* 2010, vol. 22, 17-33
- **CHODAVARAPU et al.** Transcriptome and methylome interactions in rice hybrids. *Proc. Natl. Acad. Sci. U.S.A.,* 2012, vol. 109, 12040-12045
- **ZHAO et al.** Epigenetic inheritance and variation of DNA methylation level and pattern in maize intra-specific hybrids. *Plant Science,* 2007, vol. 172, 930-938

- **ZHANG et al.** Endosperm-specific hypomethylation, and meiotic inheritance and variation of DNA methylation level and pattern in sorghum (Sorghum bicolor L.) inter-strain hybrids. *Theor. Appl. Genet.,* 2007, vol. 115, 195-207
- **LISTER et al.** Highly integrated single-base resolution maps of the epigenome in Arabidopsis. *Cell,* 2008, vol. 133, 523-36
- **VAN WOLFSWINKEL ; KETTING.** The role of small non-coding RNAs in genome stability and chromatin organization. *J. Cell Sci. 2010,* 2010, vol. 123, 1825-39
- **BARTEL.** MicroRNAs: Genomics, biogenesis, mechanism, and function. *Cell,* 2004, vol. 116, 281-297
- **KHRAIWESH et al.** Role of miRNAs and siRNAs in biotic and abiotic stress responses of plants. *Biochim. Biophys. Acta,* 2012, vol. 1819, 137-48
- **MALLORY ; VAUCHERET.** Functions of microRNAs and related small RNAs in plants. *Nat. Genet.,* 2006, vol. 38, S31-S36
- **BARBER et al.** Repeat associated small RNAs vary among parents and following hybridization in maize. *Proc. Natl. Acad. Sci. U.S.A.,* 2012, vol. 106, 10444-10449
- **NOBUTA et al.** Distinct size distribution of endogeneous siRNAs in maize: Evidence from deep sequencing in the mop1-1 mutant. *Proc. Natl. Acad. Sci. U.S.A.,* 2008, vol. 105, 14958-14963
- **SHEN et al.** Genome-Wide Analysis of DNA Methylation and Gene Expression Changes in Two Arabidopsis Ecotypes and Their Reciprocal Hybrids. *The Plant Cell,* 2012, vol. 24, 875-892
- **SCHOLTEN et al.** hypothesize that small non-coding RNAs have a prediction potential. *Diagnostics in plant breeding,* 01 January 2013, 265ff
- **WINDHAUSEN et al.** Effectiveness of genomic prediction of maize hybrid performance in different breeding populations and environments. *G3,* 2012, vol. 2, 1427-1436
- Genetic diversity and heterosis. **MELCHINGER.** The genetics and exploitation of heterosis in crops. ASA-CSSA, 1999, 99-118
- **SCHRAG et al.** Prediction of single-cross hybrid performance for grain yield and grain dry matter content in maize using AFLP markers associated with QTL. *Theor. Appl. Genet.,* 2006, vol. 113, 1037-1047
- **SCHRAG et al.** Molecular marker-based prediction of hybrid performance in maize using unbalanced data from multiple experiments with factorial crosses. *Theor. Appl. Genet.,* 2009, vol. 118, 741-751
- **WINDHAUSEN et al.** Effectiveness of Genomic Prediction of Maize Hybrid Performance in Different Breeding Populations and Environments. *G3,* 2012, vol. 2, 1427-1436
- **REIF et al.** Genomic prediction of sunflower hybrid performance. *Plant Breed,* 2012, vol. 132, 107-114
- **GÄRTNER et al.** Improved Heterosis Prediction by Combining Information on DNA- and Metabolic Markers. *PLOS ONE,* 2009, vol. 4, e5220
- **RIEDELSHEIMER et al.** Genomic and metabolic prediction of complex heterotic traits in hybrid maize. Nature Publishing Group, 2012, vol. 44, 217-220
- **HOCHHOLDINGER ; HOECKER.** Towards the molecular basis of heterosis. *Trends in Plant Science,* 2007, vol. 12, 427-432
- **FU et al.** Partial least squares regression, support vector machine regression, and transcriptome-based distances for prediction of maize hybrid performance with gene expression data. *Theor. Appl. Genet.,* 2012, vol. 124, 825-833
- **FRISCH et al.** Transcriptome-based distance measures for grouping of germplasm and prediction of hybrid performance in maize. *Theor. Appl. Genet.,* 2010, vol. 120, 441-450
- **STOKES et al.** An association transcriptomics approach to the prediction of hybrid performance. *Mol. Breeding,* 2010, vol. 26, 91-106
- **SHIVAPRASAD et al.** Extraordinary transgressive phenotypes of hybrid tomato are influenced by epigenetics and small silencing RNAs. *The EMBO Journal,* 2011, vol. 31, 257-266
- **BARBER et al.** Repeat associated small RNAs vary among parents and following hybridization in maize. *Proc. Natl. Acad. Sci. U.S.A.,* 2012, vol. 109, 10444-10449
- **MAXAM ; GILBERT.** A new method for sequencing DNA. *Proc. Natl. Acad. Sci. U.S.A.,* 1977, vol. 74, 560-564
- **SANGER ; COULSON.** A rapid method for determining sequences in DNA by primed synthesis with DNA polymerase. *Journal of Molecular Biology,* 1975, vol. 94, 441-448
- **VARKONYI-GASIC et al.** Protocol: a highly sensitive RT-PCR method for detection and quantification of microRNAs. *Plant Methods,* 2007, vol. 3, 12
- **BOWTELL ; SAMBROOK.** DNA microarrays: a molecular cloning manual. Cold Spring Harbor Laboratory Press, 2003
- **MCCORMICK et al.** Experimental design, preprocessing, normalization, and differential expression analysis of small RNA sequencing experiments. *Silence,* 2011, vol. 2, 2
- **BOLSTAD et al.** A comparison of normalization methods for high density oligonucleotide array data based on variance and bias. *Bioinformatics,* 2003, vol. 19, 185-93
- **BENJAMINI ; HOCHBERG.** Controlling the false discovery rate: a practical and powerful approach to multiple testing. *J. R. Statist. Soc. B,* 1995, vol. 57, 289-300